Europäisches Patentamt

European Patent Office

Office européen des brevets

(11) Publication number: **0 272 407**
**A2**

# EUROPEAN PATENT APPLICATION

(21) Application number: 87115676.6

(22) Date of filing: 26.10.87

(51) Int. Cl.⁴ **G01N 21/78** , C12Q 1/00 ,
G01N 33/52

The title of the invention has been amended (Guidelines for Examination in the EPO, A-III, 7.3).

(30) Priority: 29.10.86 JP 257388/86

(43) Date of publication of application:
29.06.88 Bulletin 88/26

(84) Designated Contracting States:
DE FR GB IT SE

(71) Applicant: KONICA CORPORATION·
No. 26-2, Nishi-shinjuku 1-chome
Shinjuku-ku Tokyo(JP)

Applicant: Chugai Pharmaceutical Co., Ltd.
5-1, Ukima 5-chome Kita-ku
Tokyo(JP)

(72) Inventor: Hamaguchi, Takehiko
c/o KONICA CORPORATION 1, Sakuramachi
Hino-shi Tokyo(JP)
Inventor: Ishihara, Takashi
c/o KONICA CORPORATION 1, Sakuramachi
Hino-shi Tokyo(JP)
Inventor: Haga, Isao
c/o KONICA CORPORATION 1, Sakuramachi
Hino-shi Tokyo(JP)
Inventor: Zanma, Souichi c/o Chugai
Pharmaceutical Co. Ltd.
41-8, Takada 3-chome
Toshima-ku Tokyo(JP)

(74) Representative: Henkel, Feiler, Hänzel &
Partner
Möhlstrasse 37
D-8000 München 80(DE)

(54) Analytical unit for optical assay.

(57) Disclosed is an analytical unit comprising an analytical element having at least one reagent layer provided on a transparent support and capable of causing a change in optical density when a specimen to be assayed is drip-applied thereon, and a photometric method capable of catching reflected light of the light incident from the side of the transparent support, wherein the analytical unit contains a conversion processing method in which a conversion equation for converting the optical reflection density of the light caught by the photometric method into at least one of the density of, and the value of enzymatic activity of, a component detected in a specimen to be assayed comprises a hyperbola.

According to this invention, the hyperbola is used as the conversion equation when the optical reflection density is converted into the substance density or value of enzymatic activity of a component detected.

## Analytical unit

## BACKGROUND OF THE INVENTION

This invention relates to an analytical unit for determining the density of, or the value of enzymatic activity of, a component detected in a specimen to be assayed, with use of an analytical device (or element), particularly, a dry analytical element.

In the analytical element of this sort, the specimen to be assayed, for example, whole blood, serum, plasma, etc. may be drip-applied thereon, whereupon it comes into contact with a reagent in the analytical element to cause a color reaction. Accordingly, light may be irradiated from the support side of an analytical element to effect photometry of the light reflected from the analytical element, so that the degree of the color reaction can be detected. and this degree can be related to the density of, or the value of enzymatic activity, of a specific component, i.e., a component detected in the specimen to be assayed.

However, the degree of the color reaction in the reagent can be, in general, proportional to the optical transmission density of transmitted light, but can not have no linear relationship with the optical reflection density of reflected light.

Accordingly, when based on the optical reflection density, any conversion equation is required in converting the optical reflection density into the density of a substance or the value of enzymatic activity.

This conversion equation is available in "Method for Spectral Measurement in Clinical Biochemical Assay" (edited by Kazuo Yoshino and Hisao Ohsawa, published by Gakkai Shuppan Center), in which it is known that in an analytical unit employing a sheet of color test paper, the relationship between the reflectance R and the substance density can be determined by Kubelka-Munk's equation (1):

$$\frac{K}{S} = \frac{(1-R)^2}{2R} \qquad (1)$$

In the equation, S represents a scattering coefficient, K represents an absorption coefficient, and K.S is regarded as being proportional to the substance density.

On the other hand, there are also taught in this literature those based on Williams-Clapper's equation and Beer's law.

However, the above known conversion equation can not be applied as it is to the case where the analytical element targeted in this invention is used and particularly where the light is irradiated from the side of a support of a reagent layer to effect photometry of the reflected light.

In other words, the above known conversion equation can not be applied for the reasons that (1) the reflection may occur on the surface of the light-incident side of a transparent support, (2) some of analytical elements may have a reflective layer, which, however, can not serve as a perfect reflective layer, and (3) if an aperture for the light incident on an analytical element is provided with a dust glass to prevent dust from invading a light-incident means and light-receiving (photometry) means, the surface reflection may occur on the surface of the light-incident side of the dust glass.

It is also likely that the above conversion equation can not answer to the case when there is a difference in the measurement wavelength or measuring items.

In addition, the known conversion equation, which is an equation containing a quadric function. a higher degree function or an exponent, is so much complicated that it can not be suited for the case when a simple and rapid processing is intended. Namely, the conversion equation is so complicated that an arithmetic unit of a great capacity is required, resulting in a high cost. For all that, the processing speed may be lowered if the capacity is made smaller.

As mentioned above, in the prior art. it is difficult to obtain a general equation which can be common to many items for the components detected, and particularly difficult to obtain a general equation which can be common to both of the end point assay and rate assay. Therefore, there must be applied respectively separate general equations, to which. however, a simplified analytical unit can not answer, raising another problem.

Moreover, making a close investigation, although the above conversion equation can be substantially appropriate when applied under a certain measurement wavelength and for certain measuring items, there can not be seen a complete identity between the optical density and the substance density or the value of enzymatic activity over the whole dynamic range of the components detected. Accordingly, the accuracy at a high-value region may be lost in the case of a calibration curve laying stress on a low-value region, and, on the contrary, the low-value region may become inaccurate when laying stress on the high-value region.

On the other hand, if the conversion equation is complicated as mentioned above or a same conversion equation can not be used at the time of correcting the error originating from an analytical element and/or an analytical unit by using a reference specimen having a reference (known) substance density or value of enzymatic activity, it follows that a great time is required for the correction.

## SUMMARY OF THE INVENTION

Accordingly, an object of this invention is to provide, in an analytical unit employing an analytical element, an analytical unit that can make conversion according to a simple conversion equation capable of readily carrying out arithmetic processing when the reflection density is converted into the substance density or value of enzymatic activity, can readily correct the error depending on the measurement wavelength or the difference in the calibration curve due to the difference in the measurement items or analytical element, and can have a conversion means having a high precision and accuracy over the whole dynamic range of the components detected.

Another object of this invention is to provide, in an integral type analytical unit having the above characteristic features and also capable of making the measurement according to both the end point assay and rate assay, an inexpensive analytical unit constituted of a simple arithmetic processing system, that can make conversion according to a same conversion general equation for both of the assays.

The above object can be achieved by an analytical unit comprising an analytical element having at least one reagent layer provided on a transparent support and capable of causing a change in optical density when a specimen to be assayed is drip-applied thereon, and a photometric means capable of catching reflected light of the light incident from the side of said transparent support, wherein said analytical unit contains a conversion processing means in which a conversion equation for converting the optical reflection density of the light caught by said photometric means into at least one of the density of, and the value of enzymatic activity of, a component detected in a specimen to be assayed comprises a hyperbola.

## BRIEF DESCRIPTION OF THE DRAWING

Fig. 1 is a partially cut-away sectional view illustrating the relationship between an analytical element and a photometric means;

Fig. 2 is a schematic view of the whole of an analytical unit;

Fig. 3 is an explanatory view showing an example for the layer constitution of an analytical element and a state of the reflection of incident light;

Fig. 4 is a view showing the correlation between a reflection spectrum and a measurement wavelength;

Fig. 5 is an explanatory view for the end point assay;

Fig. 6 is an explanatory view for the rate assay;

Fig. 7 is a flow chart showing a measuring method;

Figs. 8A and 8B are correlation views showing the results of examining the appropriateness about Glu according to a conversion equation;

Figs. 9A and 9B are correlation views concerning GOT;

Figs. 10 and 11 are correlation views concerning GPT and BUN, respectively;

Figs. 12A to 12C and Figs. 13A to 13C are views showing the correlations in the cases of the logarithmic regression about GPT and BUN, respectively.

## DESCRIPTION OF THE PREFERRED EMBODIMENTS

This invention will be described below in detail.

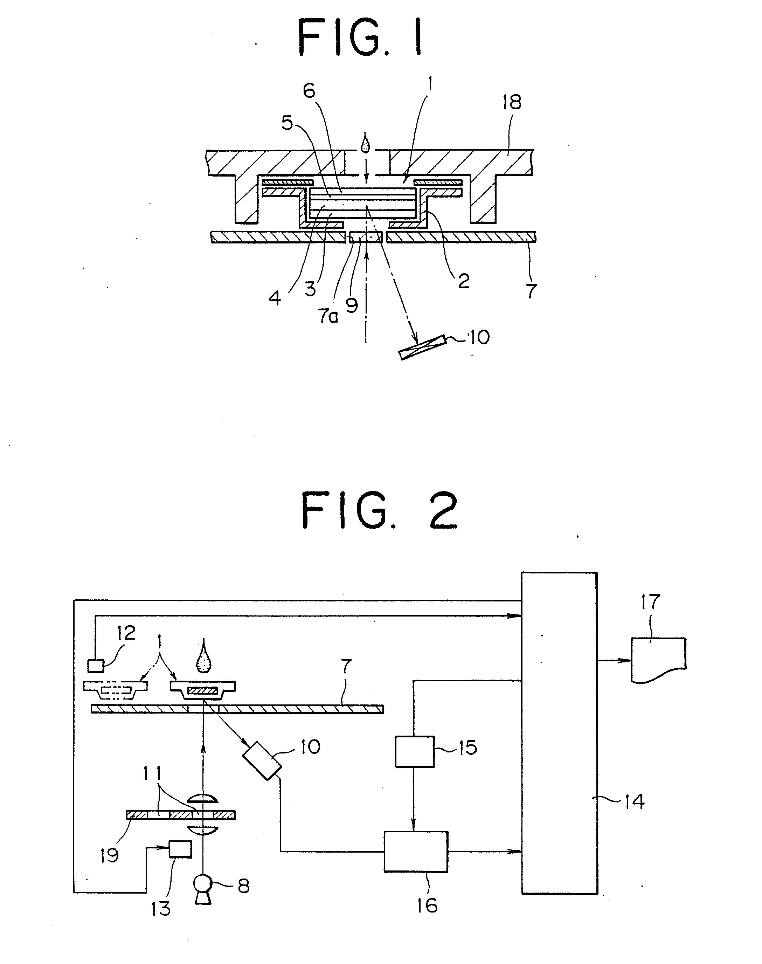

Explaining first an example of the unit of this invention on the bases of Fig. 1 to Fig. 3, the numeral 1 denotes an analytical device (or element). In a case 2, its bottom portion is comprised of a transparent support 3 made of a light-transmissive plastic or the like, and a reagent layer (a coloring layer) 4, a reflective layer 5 and a spreading layer 6 are provided on the support. The case 2 is provided with an

3

opening at its bottom portion and placed on a table 7, and a light source is provided at a lower position of an aperture 7a of the table 7. A dust glass 9 is optionally fitted to the aperture 7a in order to prevent dust from invading a photometric system, i.e., the lower part of the table 7. At the lower part of the table 7, further provided is a light-receiving sensor 10 as a photometric means, watching the aperture of the analytical element.

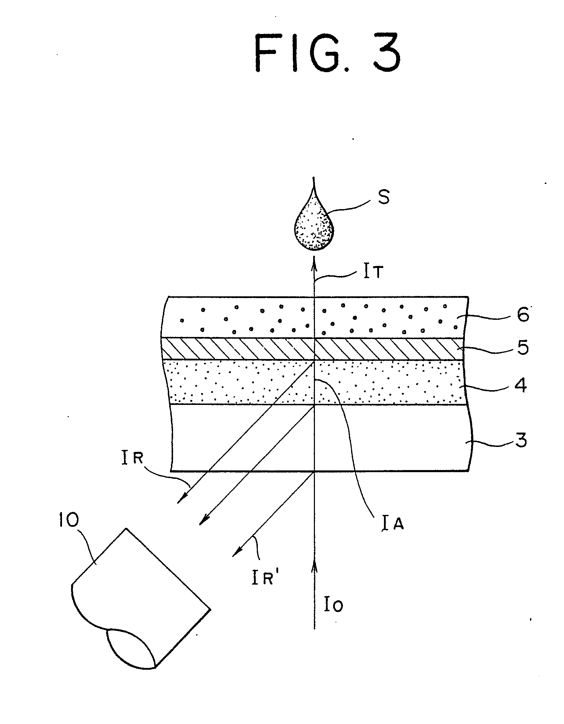

In such a unit, the light from the light source 8 passes through a filter 11 adapted to the measurement wavelength, and is made incident on the analytical element 1 through the dust glass 9. The light incident thereon is reflected in part on the surface of the transparent support 3 as shown in Fig. 3, and also reflected on the interface between layers 3 and 4 and on the interface between layers 4 and 5, the remaining being transmitted. Here, the amount of the incident light is assumed as $I_O$; the amount of the surface-reflected light, as $I_R'$; the amount of the reflected light on the interface, as $I_R$; and the amount of the transmitted light, as $I_T$.

In this way, a specimen S to be assayed is dropped on a reagent section, whereupon, while spreading over a spreading layer 6, it passes through a reflective layer 5 and enters into a reagent layer 4. A color reaction takes place there. By this reaction, the degree of reflection against the incident light changes, and chiefly $I_R$ and $I_R'$ are caught by a light-receiving sensor, whereby the substance density or value of enzymatic activity of a component detected in the specimen to be assayed can be determined on the basis of the conversion equation described hereinbelow.

The whole construction of the analytical unit is as shown in Fig. 2. Indications attached to the analytical element 1 having been set on the table 7 are read by means of a read-out sensor 12 to grasp the measuring items and measuring methods. Thereafter, the filter is rotated to a measuring position by driving and rotating a drive motor 13 for a filter-rotating disc 19 on the basis of a command from a measuring device 14 containing an arithmetic unit. Subsequently, light is irradiated on the analytical element 1 to effect photometry by means of the light-receiving sensor 10. The signals thus obtained are converted into digital signals through an AD converter 16 on the basis of a timing command from a timing generator 15, and taken into the arithmetic unit of the measuring device 14 to effect arithmetic conversion processing on the basis of the conversion equation described below. The results obtained are indicated on an indicator 17 comprising a printer or the like.

Now, in this invention, a main feature is to use a hyperbola represented by equation (2) when the photometric value (optical reflection density) caught by the photometric means is converted into an analytical value comprising the substance density or the value of enzymatic activity.

$$Y = \frac{B}{X - A} + C \qquad (2)$$

In equation (2), Y represents the substance density or value of enzymatic activity of a component detected, and X is determined by optical reflection density $D_R$ in the case of the end point assay or, in the case of the rate assay, by the change with time in the reaction density. For example, in the case of the two point photometry, it represents the rate of change in the reflection density per unit time, i.e., $\Delta D_R \cdot \Delta t$. Also, A, B and C each represent a constant determined by the type of the analytical element or a characteristic value inherent in the analytical unit. In the case of the rate assay, it is also possible to assume as X the value relating to the reaction speed based on the definitions for the rate assay.

When according to this invention the conversion equation for converting the optical reflection density into an analytical value (the substance density and/or value of enzymatic activity of a component detected) comprises the hyperbola represented by equation (2), it is possible, as in examples described below, to obtain a calibration curve with high precision and accuracy over the whole dynamic range of the components detected.

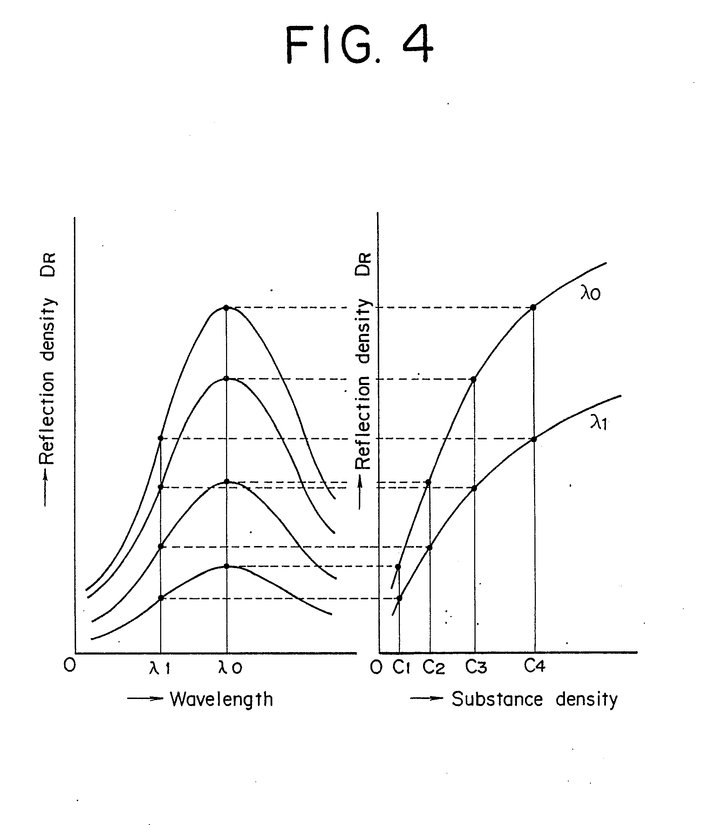

Fig. 4 shows the relationship between the reflection spectrum and measurement wavelength of a colored analytical element. In general, selected as the measurement wavelength is the wavelength $\lambda_c$ which shows the maximum value of the reflection spectrum. However, in the region of a high substance density or the region of a high value of enzymatic activity in the case the absorbance of a dye formed according to the color reaction is sufficiently high, the reflection density can not be sufficiently increased with an increase in the substance density or value of enzymatic activity, or it may bring about a limitation of the number of the filter to select the measurement wavelength $\lambda_c$ individually showing the maximum absorption value in respect of many items for the components detected. For these reasons, it may sometimes occur that the wavelength $\lambda'$ other than the wavelength showing the maximum absorption is selected for the measurement

4

wavelength. Even in such an instance, the hyperbola of the above equation (2) may be used as a conversion equation, making it possible to perform the conversion with high precision and accuracy over the whole dynamic range of the components detected.

As will be immediately understood from equation (2), the denominator of the conversion equation is an equation of the first degree, and not an equation of the second or higher degree. There also is contained no exponential function or the like, resulting in a very simple equation. Moreover, since the same conversion general equation can be used for the analysis of the various components detected, and the hyperbola can be used as a conversion equation for both the end point assay and rate assay, it is well possible to perform rapid processing even if the arithmetic processing unit may have a small capacity.

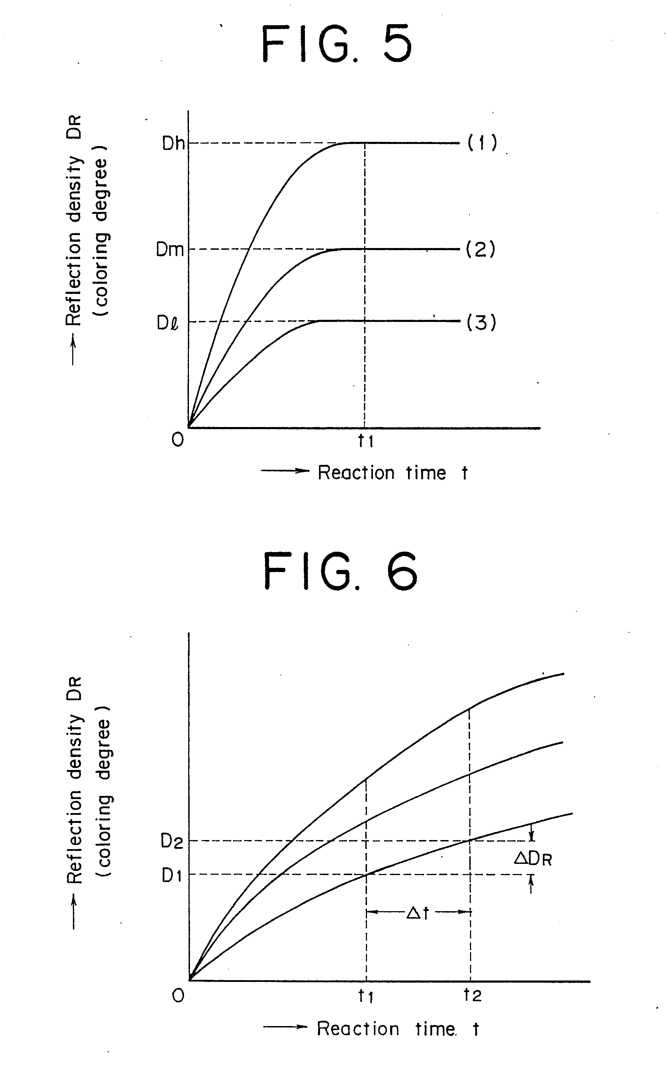

Referring next to the end point assay and rate assay, Fig. 5 and Fig. 6 show the relationship between the reaction time and coloring degree (reaction density $D_R$) in the end point assay and the rate assay, respectively. In the end point assay shown in Fig. 5, each of the reflection density $D_h$, $D_m$ and $D_l$ of (1) the sample with high substance density, (2) sample with medium substance density and (3) sample with low sabstance density at the end point $t_1$ of the reaction is used as X in the above equation (2). In the case of the rate assay shown in Fig. 6, the gradient of the time between $t_1$ and $t_2$ corresponding to the region in which the reaction is stable is used as X in the above equation (2). Specifically, in the case of the two point photometry between $t_1$ and $t_2$, $\Delta D_R / \Delta t = (D_2 - D_1)/(t_2 - t_1)$ may be used as the gradient, and, in order to further increase the precision, it is also possible to effect multi-point photometry between $t_1$ and $t_2$ and determine an average gradient between $t_1$ and $t_2$ from the data obtained. Meanwhile, $t_1$ and $t_2$ are selected by keeping out of an unstable region at the initial stage of the reaction, or from a region from which the influence by co-existent substances or the like has been eliminated.

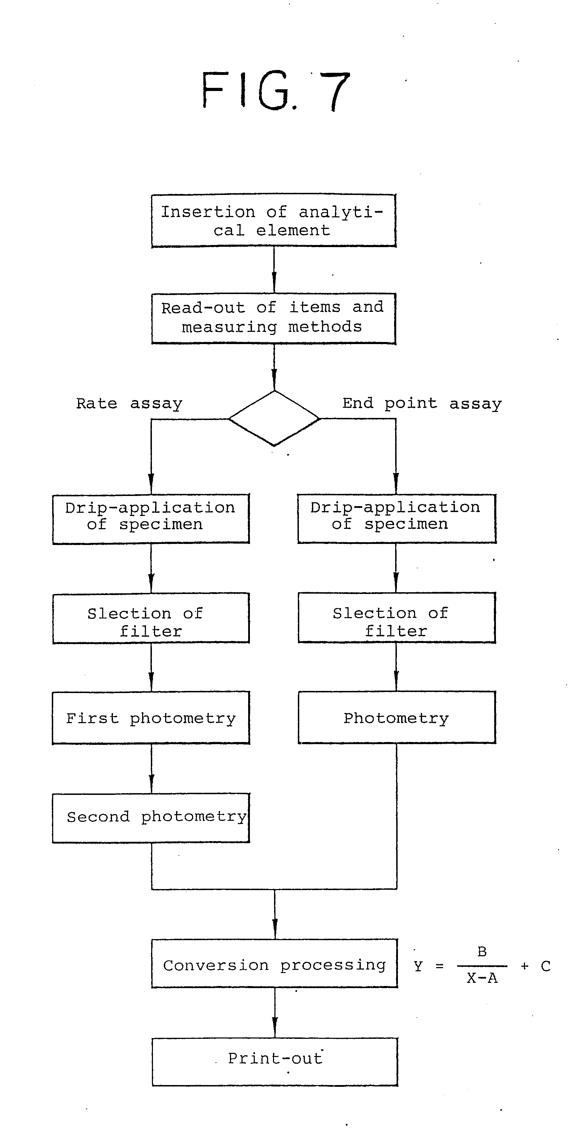

Fig. 7 is a flow chart of the measuring method.

The present inventors have made studies on whether the hyperbola can be appropriate as a conversion equation in respect of various components detected. As a result, they have found that it is very appropriate as explained below.

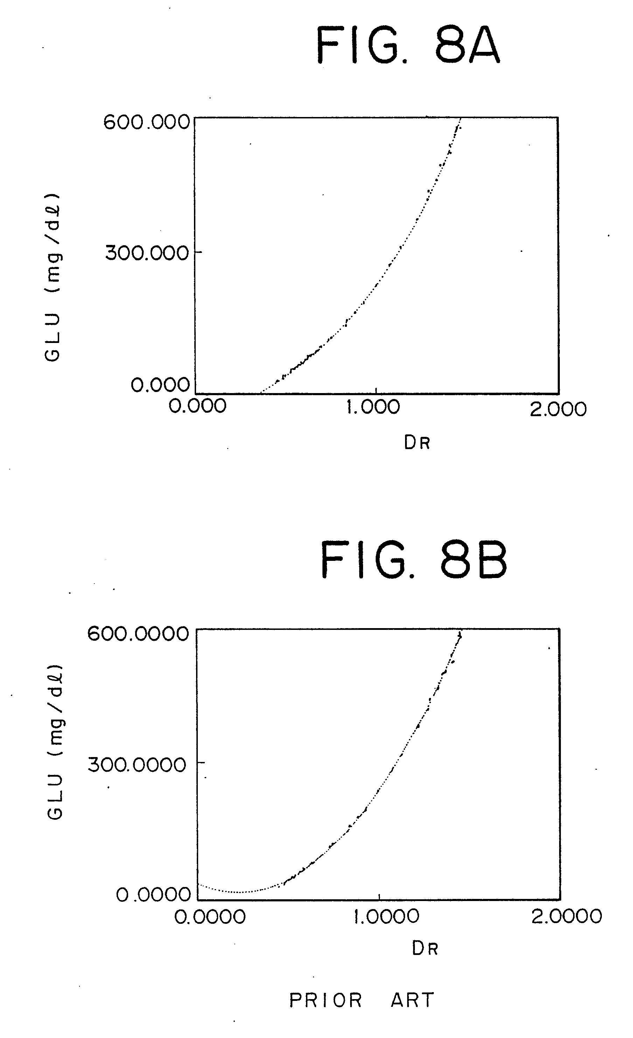

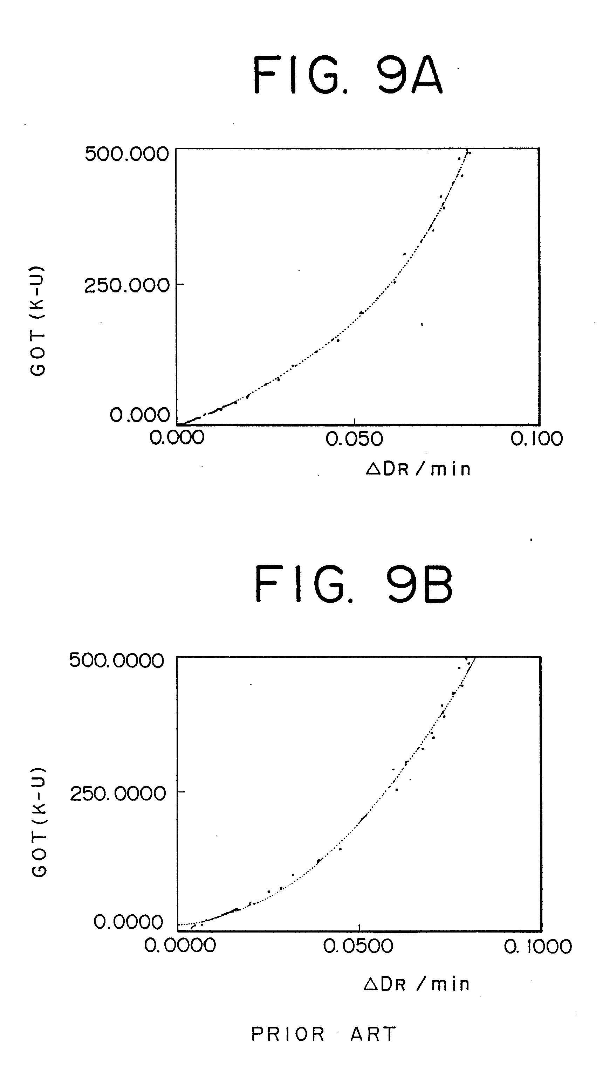

Fig. 8 and Fig. 9 show examples of actual measurement according to the end point assay and rate assay, respectively. The analytical unit used is of an integral type that can perform both the end point assay and rate assay, and provided with the dust glass between the analytical element and photometric part.

Regarding Figs. 8A and 8B, there is used an analytical element for analysis of glucose (Glu) as an example of the measurement according to the end point assay. Fig. 8A shows an example of the measurement where the hyperbola according to this invention is used as the conversion equation; and Fig. 8B, the measurement where a curve of secondary degree is used as the conversion equation as an example of the prior art.

It is understood from these Figures that the normal value region ranges between 60 and 100 mg/dl, and, although there is no difference between the both at the highly abnormal region of 100 to 600 mg/dl exceeding that region and there is also seen no great difference in the correlation coefficient, a deviation downward from the calibration curve is seen in the vicinity of the normal value region generally in the case of the regression of the curve of secondary degree in Fig. 8B, resulting in the giving of an analytical value somewhat higher than the actual value as for the substance density.

Regarding Figs. 9A and 9B, there is used an analytical element for analysis of glutamate-oxaloacetate transaminase (GOT) as an example of the measurement according to the rate assay. Fig. 9A and Fig. 9B show examples of the measurement where the hyperbola and the curve of secondary degree are respectively used as the conversion equations. Similar to the case of Glu, there is no great difference between the both at the highly abnormal region of 50 to 500 Karmen Unit (K-U), but a great deviation from the calibration curve is seen in the vicinity of the normal value region of 5 to 50 K-U.

The relationship of these is shown in Table 1. The "error" shown in Table 1 indicates a value of the standard deviation of the degree of deviation from the calibration curve. With regard to both Glu and GOT, there is seen a great error in the vicinity of the normal value in the case of the regression of the curve of secondary degree, as compared with the case of the hyperbola regression.

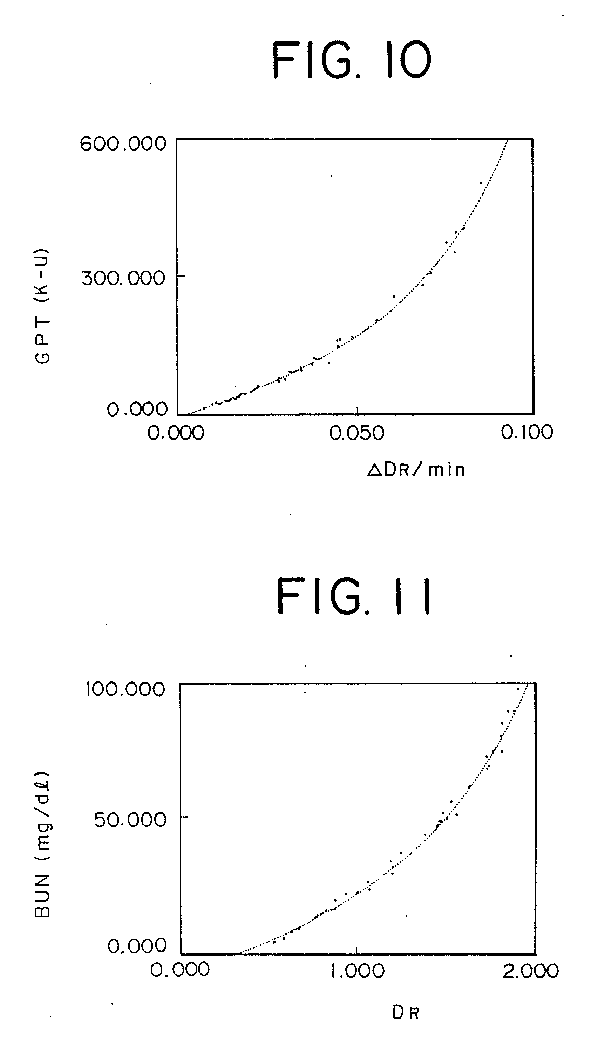

Fig. 10 shows an example of the hyperbola regression of the calibration curve in respect of glutamic-pyruvic transaminase (GPT) according to the rate assay; and Fig. 11, in respect of blood urea nitrogen (BUN) according to the end point assay. It is understood in both cases that accurate correlations are seen over the whole dynamic range.

Similarly, appropriate correlations were obtained in respect of other components detected.

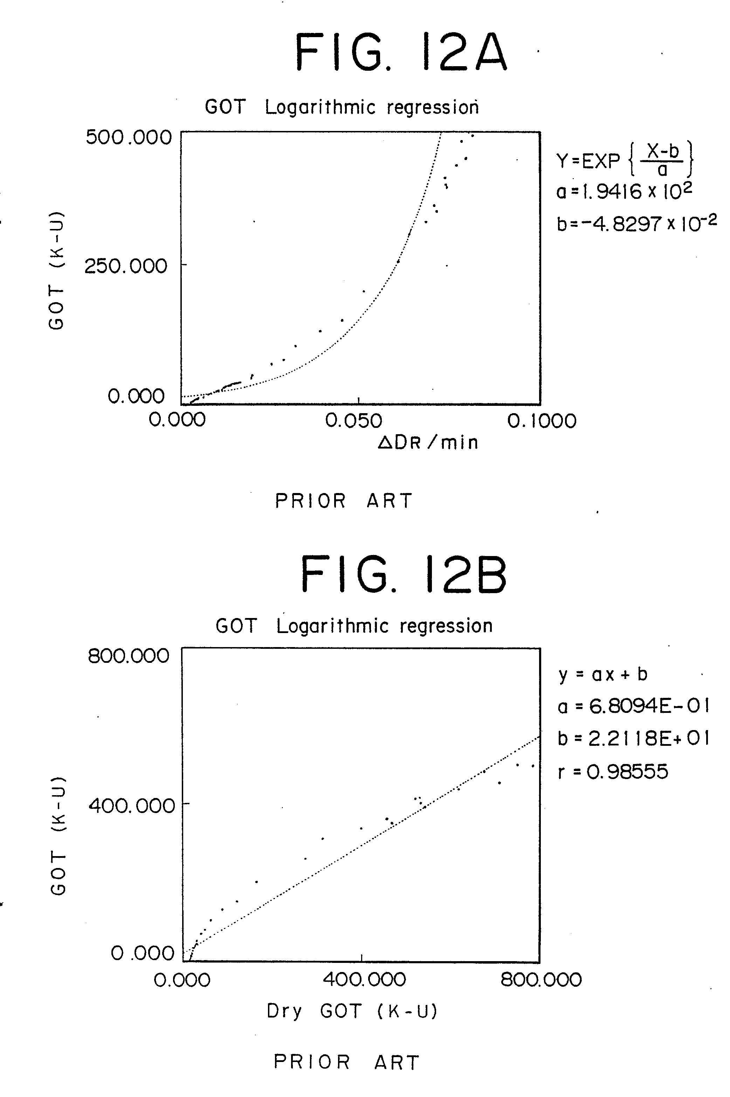

On the other hand, the appropriateness of the logarithmic regression was also examined about GOT. Results obtained are shown in Figs. 12A to 12C. The measuring method is in accordance with the rate assay.

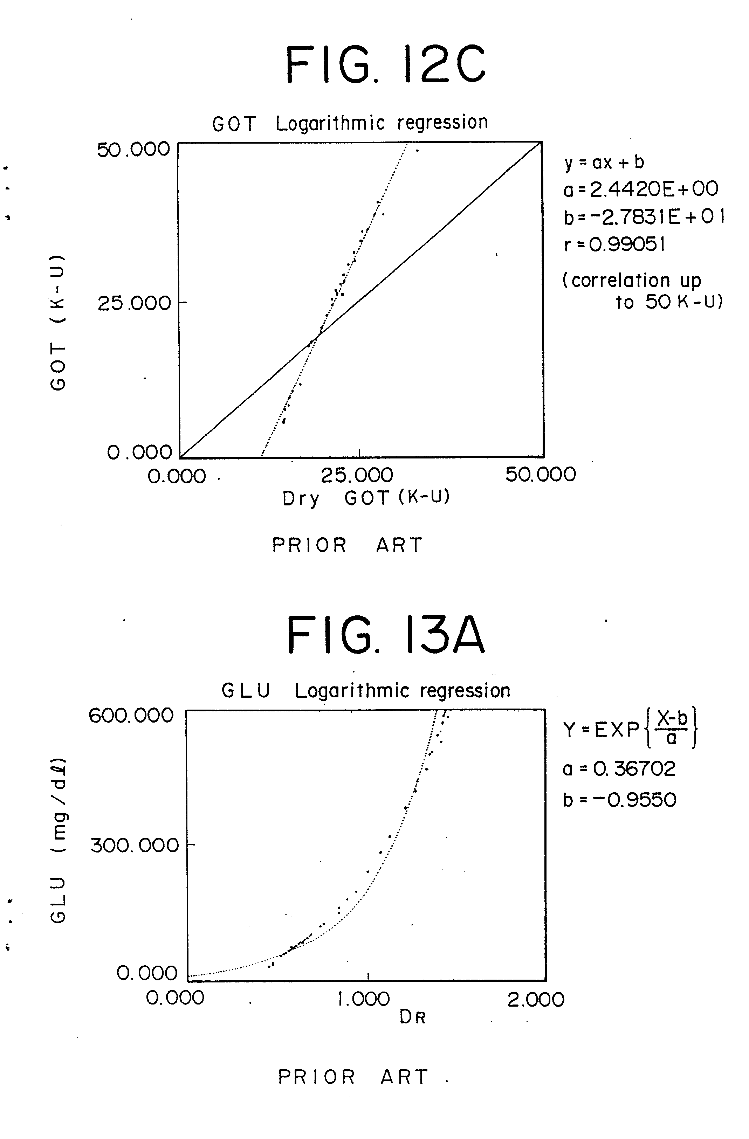

It is understood from Fig. 12A that there is a considerably great deviation. It is also understood that as shown in Fig. 12B and Fig. 12C. any accurate correlation with the standard measured value can not be obtained according to the logarithmic regression. It is understood that such a great deviation can not be corrected at all.

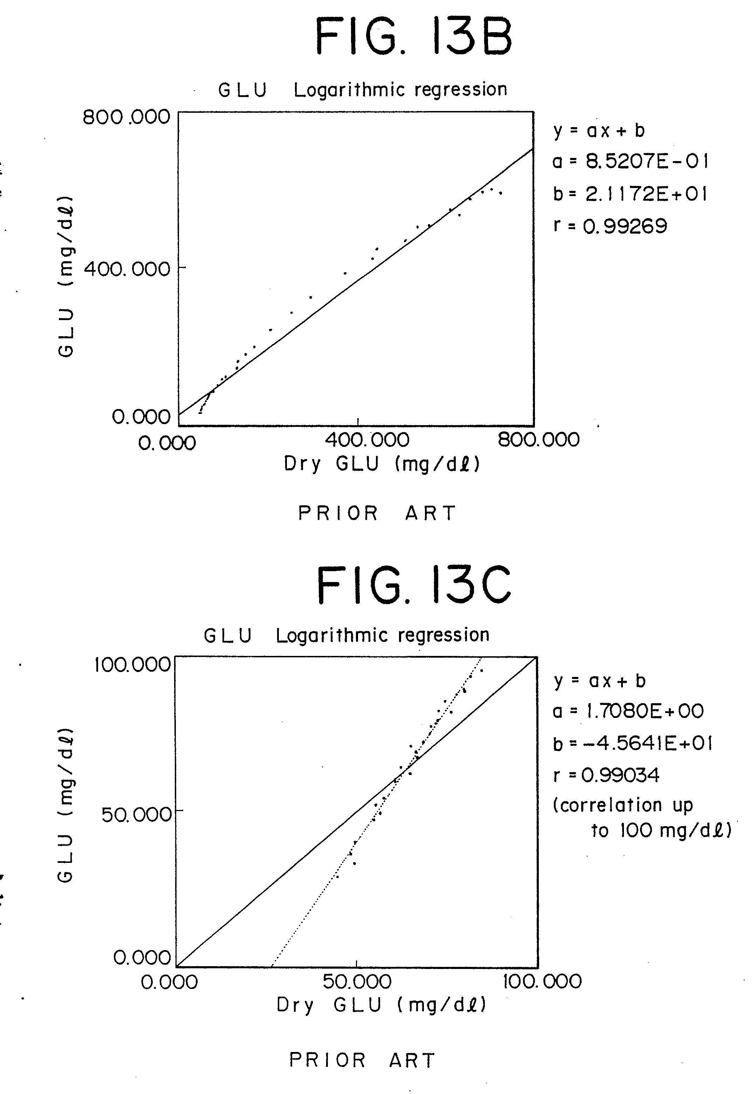

Figs. 13A to 13C show the results of the logarithmic regression in respect of Glu according to the end point assay. It is understood that the results are similar to those of the logarithmic regression in respect of GOT.

In the above, references were made on the quadratic or logarithmic conversion equation as the prior arts. but it was found that the results are considerably accurate in the case of the third or higher degree regression.

However, for example, in the case of the third degree regression, there are involved four variables, and the variables may increase with increase to the fourth degree and fifth degree, resulting in no possibility at all for use in a simple analysis. Also, as a result, the kind of the specimen to be assayed must be increased at the time of the correction, and, because of an increase in the points of inflection, the calibration curve may take the form of a complicated curve to make it very difficult to form the calibration curve.

As described above, according to this invention, the hyperbola is used as the conversion equation when the optical reflection density is converted into the substance density or value of enzymatic activity of a component detected. Accordingly, it is possible to eliminate the various influences arising in the reflection photometry that have been conventionally called in question, to therefore provide a calibration curve with high precision and accuracy over the whole dynamic range of the components detected, to readily make the arithmetic processing because the conversion equation is simple and the hyperbola can be applied together to various components detected. to therefore need only a small capacity for a computer used for the arithmetic processing. contained in the analytical unit, and also to readily carry out the so-called correction to correct errors originating from the analytical element and analytical unit.

It is further possible to make common and simplify the arithmetic processing section because the conversion general equation can be same even in the integral type analytical unit capable of carrying out the measurement according to the end point assay and rate assay.

Table 1

| Compo-nent detect-ed | Conversion equation | Correlation over the whole dynamic range | | | Correlation in the vicinity of normal value | | | Error | Constant |
|---|---|---|---|---|---|---|---|---|---|
| | | Range | n | Coef-ficient of cor-relation | Range | n | Coef-ficient of cor-relation | | |
| Glu | Hyperbola $Y = \dfrac{B}{X - A} + C$ | 30-600mg/dℓ | 50 | 0.9996 | 30-100mg/dℓ | 28 | 0.9987 | 2.12 mg/dℓ | A= $2.458 \times 10^{0}$ B=$-1.127 \times 10^{3}$ B=$-5.341 \times 10^{2}$ |
| | Curve of secondary degree $Y = AX^{2} + BX + C$ | 30-600mg/dℓ | 50 | 0.9994 | 30-100mg/dℓ | 28 | 0.9732 | 4.10 mg/dℓ | A= $3.760 \times 10^{2}$ B=$-1.739 \times 10^{2}$ C= $4.045 \times 10^{1}$ |
| GOT | Hyperbola $Y = \dfrac{B}{X - A} + C$ | 5-500KU | 50 | 0.9991 | 5- 50KU | 30 | 0.9924 | 1.02 KU | A= $1.348 \times 10^{-1}$ B=$-4.548 \times 10^{1}$ C=$-3.424 \times 10^{2}$ |
| | Curve of Secondary degree $Y = AX^{2} + BX + C$ | 5-500KU | 50 | 0.9984 | 5- 50KU | 30 | 0.9623 | 3.16 KU | A= $6.699 \times 10^{4}$ B= $4.162 \times 10^{2}$ C= $1.132 \times 10^{1}$ |

0 272 407

## Claims

1. An analytical unit comprising an analytical element having at least one reagent layer provided on a transparent support and capable of causing a change in optical density when a specimen to be assayed is drip-applied thereon, and a photometric means capable of catching reflected light of the light incident from the side of said transparent support, wherein said analytical unit contains a conversion processing means in which a conversion equation for converting the optical reflection density of the light caught by said photometric means into at least one of the density of, and the value of enzymatic activity of, a component detected in a specimen to be assayed comprises a hyperbola.

2. The analitical unit according to Claim 1, wherein the conversion equation is:

$$Y = \frac{B}{X - A} + C$$

where X denotes the optical reflection density, Y denotes the density of, or the value of enzymatic activity of, a component detected.

3. The analytical unit according to Claim 1, wherein the conversion equation used when the density of, and the value of enzymatic activity of, said component detected is determined according to an end point assay and a rate assay is based on the hyperbola in both the methods.

4. The analitical unit according to the Claim 1, wherein the conversion processing means comprising a measuring device containing an arithmetic unit, a timing generator and an AD converter.

5. The analytical unit according to Claim 1, wherein a transparent member for preventing dust to protect the photometric means is interposed between said analytical element and said photometric means.

6. The analytical unit according to Claim 1, wherein the light incident on the analitical element is the light that passes through a filter adapted to the measurement wavelength.

7. The analytical unit according to Claim 1, wherein the transparent support is light-transmissive.

8. The analytical unit according to Claim 1, wherein said analytical unit contains a read-out sensor which read indications attached to the analytical element.

# FIG. 1

# FIG. 2

# FIG. 3

FIG. 4

# FIG. 5

Reflection density DR (coloring degree)

Dh — (1)
Dm — (2)
Dl — (3)

O    t1

Reaction time t

# FIG. 6

Reflection density DR (coloring degree)

D2
D1
$\Delta D_R$
$\Delta t$

O    t1    t2

Reaction time t

# FIG. 7

```
┌─────────────────────┐
│ Insertion of analyti-│
│    cal element       │
└─────────────────────┘
           │
           ▼
┌─────────────────────┐
│ Read-out of items and│
│   measuring methods  │
└─────────────────────┘
           │
           ▼
Rate assay      ◆      End point assay
      ┌─────────┴─────────┐
      ▼                   ▼
┌──────────────┐   ┌──────────────┐
│Drip-application│  │Drip-application│
│  of specimen  │   │  of specimen  │
└──────────────┘   └──────────────┘
      │                   │
      ▼                   ▼
┌──────────────┐   ┌──────────────┐
│ Slection of  │   │ Slection of  │
│   filter     │   │   filter     │
└──────────────┘   └──────────────┘
      │                   │
      ▼                   ▼
┌──────────────┐   ┌──────────────┐
│First photometry│  │  Photometry  │
└──────────────┘   └──────────────┘
      │                   │
      ▼                   │
┌──────────────┐          │
│Second photometry│        │
└──────────────┘          │
      └─────────┬─────────┘
                ▼
```

Conversion processing  $\quad Y = \dfrac{B}{X-A} + C$

Print-out

# FIG. 8A

# FIG. 8B

PRIOR ART

# FIG. 9A

# FIG. 9B

PRIOR ART

# FIG. 10

# FIG. 11

# FIG. 12A

### GOT Logarithmic regression

$Y = EXP\left\{\dfrac{X-b}{a}\right\}$

$a = 1.9416 \times 10^2$

$b = -4.8297 \times 10^{-2}$

PRIOR ART

# FIG. 12B

### GOT Logarithmic regression

$y = ax + b$

$a = 6.8094E-01$

$b = 2.2118E+01$

$r = 0.98555$

PRIOR ART

# FIG. 12C

## GOT Logarithmic regression

$y = ax + b$

$a = 2.4420E+00$

$b = -2.7831E+01$

$r = 0.99051$

(correlation up to 50 K-U)

Y-axis: GOT (K-U), values 50.000, 25.000, 0.000

X-axis: Dry GOT (K-U), values 0.000, 25.000, 50.000

PRIOR ART

# FIG. 13A

## GLU Logarithmic regression

$$Y = EXP\left\{\frac{X-b}{a}\right\}$$

$a = 0.36702$

$b = -0.9550$

Y-axis: GLU (mg/dl), values 600.000, 300.000, 0.000

X-axis: DR, values 0.000, 1.000, 2.000

PRIOR ART

0 272 407

# FIG. 13B

GLU Logarithmic regression

y = a x + b
a = 8.5207E−01
b = 2.1172E+01
r = 0.99269

GLU (mg/dℓ) vs Dry GLU (mg/dℓ)

PRIOR ART

# FIG. 13C

GLU Logarithmic regression

y = a x + b
a = 1.7080E+00
b = −4.5641E+01
r = 0.99034
(correlation up
to 100 mg/dℓ)

GLU (mg/dℓ) vs Dry GLU (mg/dℓ)

PRIOR ART